(19) Europäisches Patentamt
European Patent Office
Office européen des brevets

(11) Veröffentlichungsnummer: **0 441 940 B1**

(12) **EUROPÄISCHE PATENTSCHRIFT**

(45) Veröffentlichungstag der Patentschrift: **03.08.94**

(21) Anmeldenummer: **90913694.7**

(22) Anmeldetag: **04.09.90**

(86) Internationale Anmeldenummer: **PCT/EP90/01477**

(87) Internationale Veröffentlichungsnummer: **WO 91/03446 (21.03.91 91/07)**

(51) Int. Cl.[5]: **C07C 22/08**, C09K 19/30, G02F 1/13, C07C 22/04, C07C 25/18, C07C 255/54, C07C 255/50, C07C 43/225

# (54) PHENYLCYCLOHEXANE UND FLÜSSIGKRISTALLINES MEDIUM.

(30) Priorität: **05.09.89 DE 3929416**
**06.09.89 DE 3929524**
**01.03.90 DE 4006314**

(43) Veröffentlichungstag der Anmeldung: **21.08.91 Patentblatt 91/34**

(45) Bekanntmachung des Hinweises auf die Patenterteilung: **03.08.94 Patentblatt 94/31**

(84) Benannte Vertragsstaaten: **DE GB**

(56) Entgegenhaltungen:
**EP-A- 0 280 902**
**EP-A- 0 318 423**
**FR-A- 2 361 353**

(73) Patentinhaber: **MERCK PATENT GmbH**
**Postfach,**
**Frankfurter Strasse 250**
**D-64271 Darmstadt(DE)**

(72) Erfinder: **HITTICH, Reinhard**
**Am Kirchberg 11**
**D-6101 Modautal 1(DE)**
Erfinder: **WÄCHTLER, Andreas**
**Goethestrasse 34**
**D-6103 Griesheim(DE)**
Erfinder: **POETSCH, Eike**
**Am Buchwald 4**
**D-6109 Mühltal(DE)**
Erfinder: **PLACH, Herbert**
**Wingertsbergstrasse 5**
**D-6100 Darmstadt(DE)**
Erfinder: **COATES, David**
**87 Sopwith Crescent**
**Merley**
**Wimborne Dorset BH21 3SW(GB)**

**EP 0 441 940 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Phenylcyclohexane der Formel I

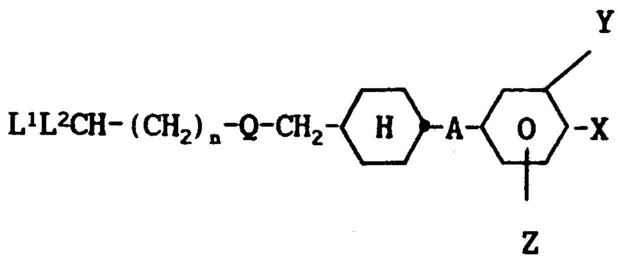

I

worin

| | |
|---|---|
| $L^1$ und $L^2$ | jeweils $CH_3$ oder einer der Reste $L^1$ und $L^2$ auch $C_2H_5$ oder $L^1L^2CH$- auch |

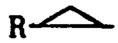 , 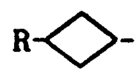 ,

| | |
|---|---|
| | wobei R H oder geradkettiges Niederalkyl mit bis zu 5 C-Atomen bedeutet, |
| n | 0 bis 5, |
| Q | -O- oder $-CH_2-$, |
| A | trans-1,4-Cyclohexylen, 1,4-Phenylen, 3-Fluor-1,4-phenylen oder eine Einfachbindung, |
| X | F, Cl, $-CF_3$, -CN, $-OCF_3$ oder $-OCHF_2$ und |
| Y und Z | jeweils unabhängig voneinander H oder F bedeuten, und diese enthaltende flüssigkristalline Medien. |

Aus der DE-OS 29 07 332 sind ähnliche Verbindungen der Formel

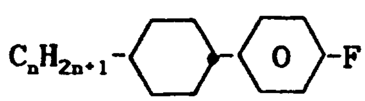

bekannt. Diese nematogenen Verbindungen eignen sich vorzüglich zur Verbesserung des Tieftemperaturverhaltens nematischer Gemische, zeigen jedoch andererseits relativ hohe Werte für den Dampfdruck für kleine Werte von n. Aus der EP-OS 02 80 902 sind ähnliche Verbindungen der Formel

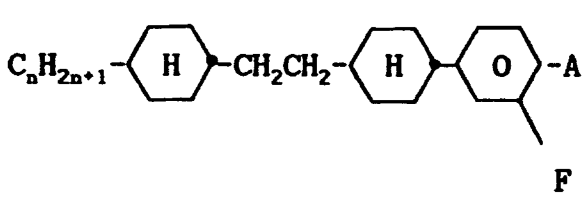

bekannt, worin A F, Cl, Br, H oder CN bedeutet. Diese Verbindungen haben zwar niedrige Werte für den Dampfdruck, jedoch andererseits deutlich smektogene Eigenschaften. Es besteht somit ein Bedarf an hoch nematogenen Verbindungen mit geringen Werten für den Dampfdruck.

Die Verbindungen der Formel I können wie ähnliche, z.B. aus der DE-OS 29 07 332 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, zu hohe Temperaturabhängigkeit der Schwellenspannung.

Insbesondere bei Anzeigen vom Supertwisttyp (STN) mit Verdrillungswinkeln von deutlich mehr als 220 ° oder bei Anzeigen mit aktiver Matrix weisen die bisher eingesetzten Materialien, z.B. die oben angegebenen Nachteile auf und haben insbesondere ungünstige elastische Eigenschaften.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, die die Nachteile der bekannten Verbindungen nicht oder nur in geringerem Maße zeigen und für die jeweilige Anwendung maßgeschneiderte elastische Eigenschaften aufweisen. Diese Aufgabe wurde durch die Bereitstellung der neuen Verbindungen der Formel I gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu sehr tiefen Temperaturen, hervorragender chemischer Stabilität, niedriger Viskosität bei positiver dielektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und relativ hohe positive dielektrische Anisotropie bei gleichzeitig günstiger Viskosität. Je nach Wahl der Parameter n und Q lassen sich die elastischen Eigenschaften in einem weiten Bereich gezielt einstellen.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität, wobei in jedem Fall auch günstige Schwellenspannungen zu realisieren sind.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische Anzeigen, die derartige Medien enthalten.

Vor- und nachstehend haben R, $L^1$, $L^2$, n, Q, A, X, Y und Z die angegebene Bedeutung, sofern nicht ausdrücklich etwas anderes vermerkt ist.

Bevorzugte Bedeutungen für $L^1L^2CH$- sind die Reste (a) bis (d):

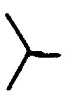
(a)

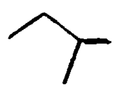
(b)

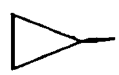
(c)

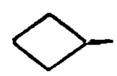
(d)

Die Reste (a) und (d) sind besonders bevorzugt. Q ist vorzugsweise $CH_2$. n ist vorzugsweise 0, 1, 2 oder 3.

Der Rest

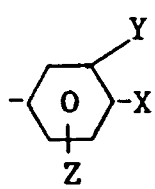

ist vorzugsweise

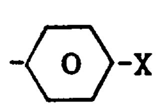,
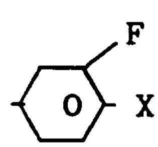
oder
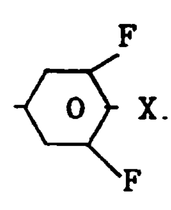.

X ist vorzugsweise F, Cl, $-CF_3$ oder $-OCF_3$.

A ist vorzugsweise eine Einfachbindung, trans-1,4-Cyclohexylen oder 1,4-Phenylen.

Bevorzugte kleinere Gruppen von erfindungsgemäßen Verbindungen entsprechen den folgenden Teilformeln:

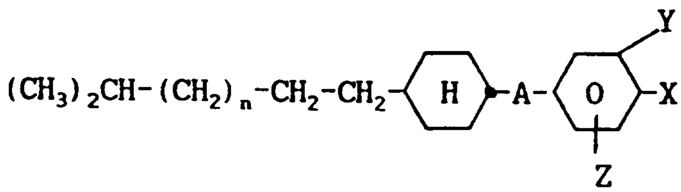

Ia

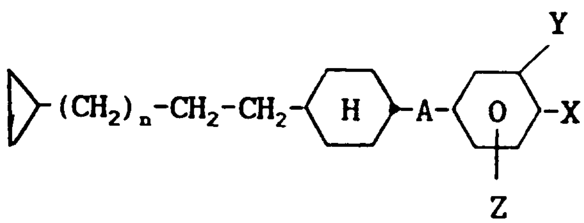

Ib

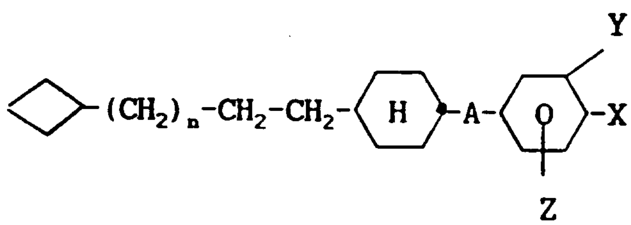

Ic

Die Verbindungen der Formel I werden im übrigen nach an sich bekannten Methoden hergestellt, wie sie in der Literatur (z.B. in den Standardwerken wie Houben-Weyl, Metho den der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise wird ein Aldehyd der Formel II

$L^1L^2CH-(CH_2)_n-CHO$ II

worin $L^1$, $L^2$ und n die angegebene Bedeutung haben mit einem Phosphoniumsalz der Formel III

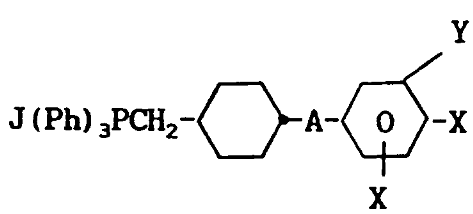

III

worin A, X, Y und Z die angegebene Bedeutung haben, nach Wittig umgesetzt. Nach Hydrierung der erhaltenen Ethenderivate an Pd-C erhält man die Verbindungen der Formel I mit $Q = CH_2$.

Die Ausgangsstoffe der Formeln II und III sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde der Formel II durch Oxydation entsprechender Alkohole oder durch Reduktion entsprechender Carbonsäuren (oder ihrer Derivate) erhältlich, die Phosphoniumsalze der Formel III nach Standardverfahren aus den entsprechenden Cyclohexylmethylalkoholen zugänglich, die ihrerseits nach folgenden Syntheseschemata erhältlich sind:

Schema 1:

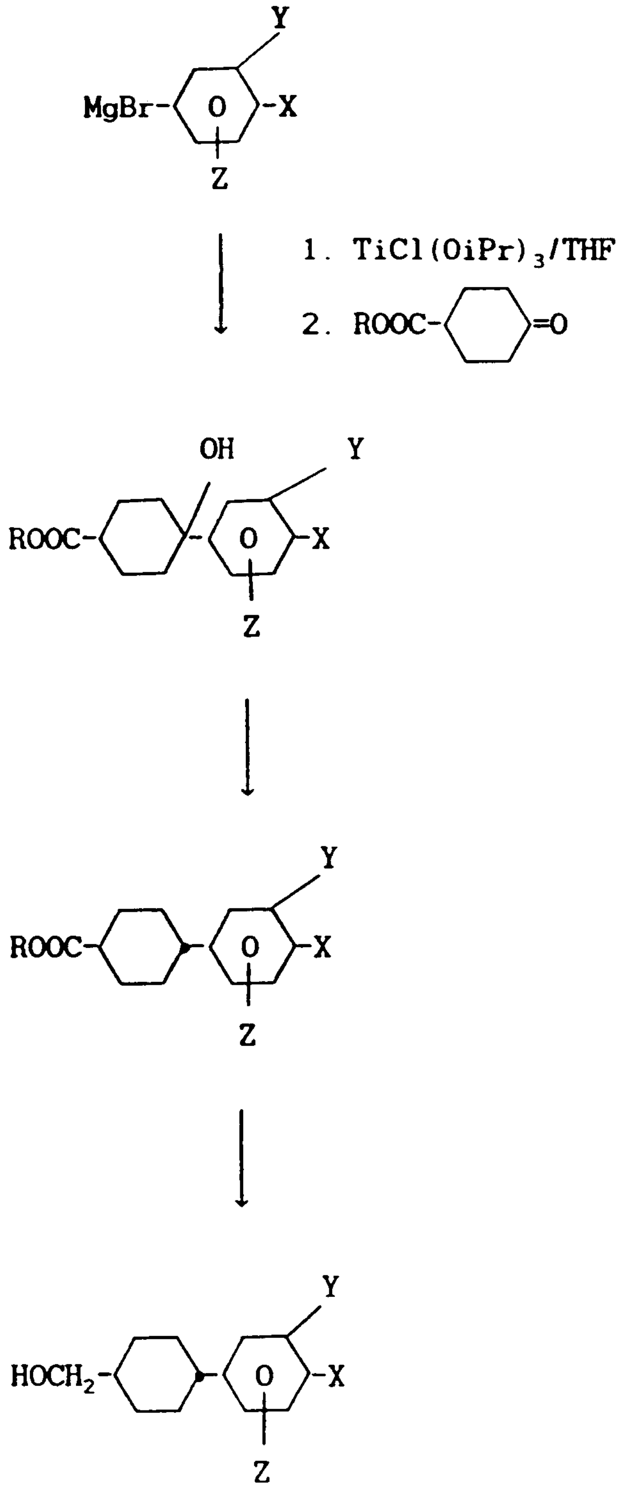

Schema 2:

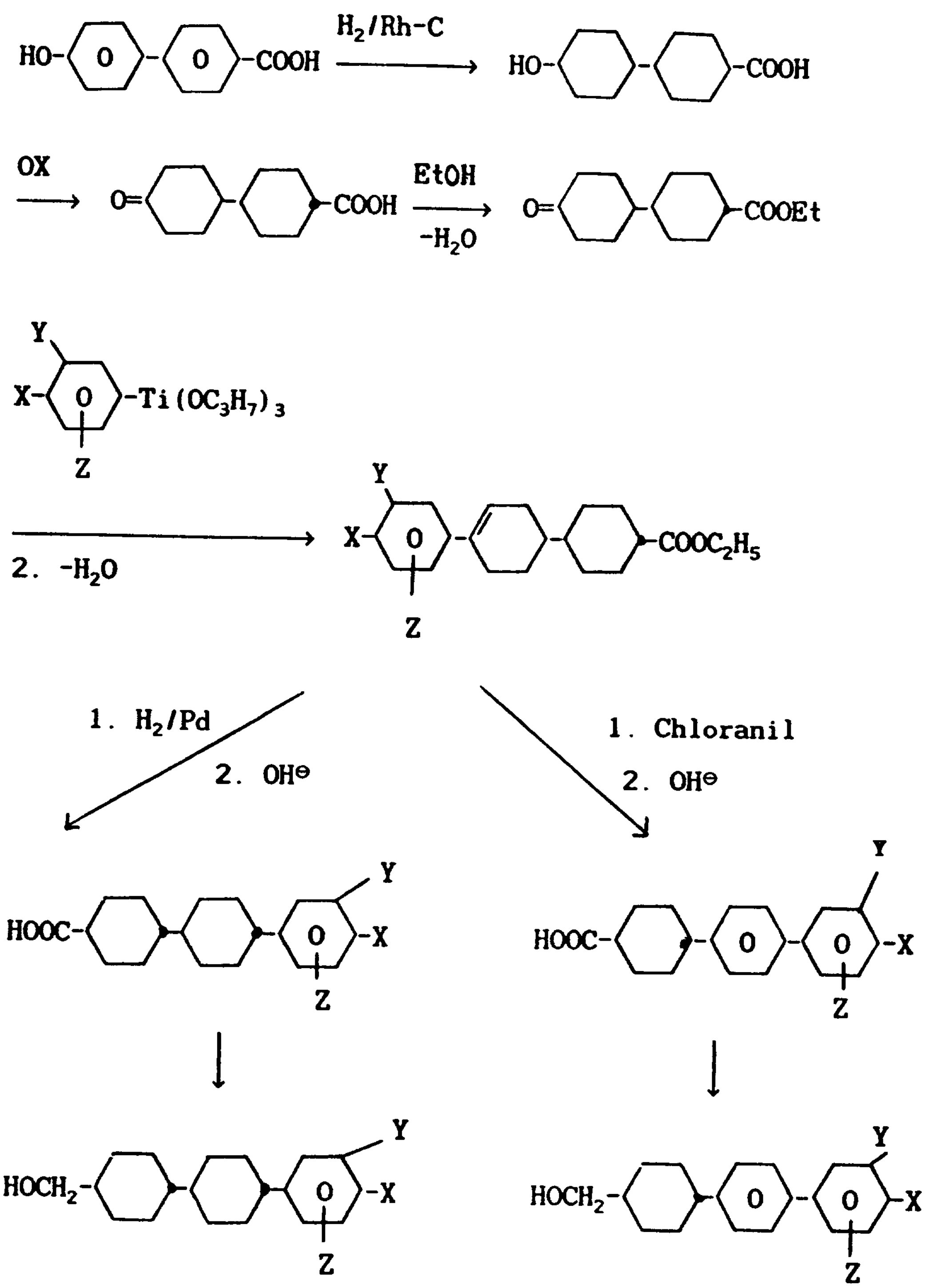


Die Verbindungen der Formel I mit A = 3-Fluor-1,4-phenylen erhält man in völliger Analogie zum ersten Syntheseschema (Herstellung von Verbindungen mit A = Einfachbindung) durch Einsatz von

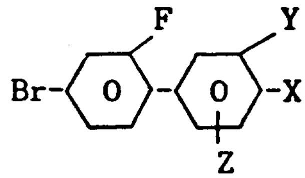

anstelle des Brombenzolderivaten. Die Brombiphenylverbindung kann in an sich bekannter Weise durch edelmetallkatalysierte Kopplungsreaktionen hergestellt werden.

Die aus dem entsprechenden Brombenzol-Derivat in Schema 1 erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohexancarbonsäureester. Aus letzterem erhält man nach üblichen Standardverfahren die geeigneten Vorprodukte für die Phosphoriumsalze.

Die Verbindungen der Formel I mit Q = O sind erhältlich durch Veretherung nach Standardmethoden der oben beschriebenen Cyclohexylmethylalkohole mit Verbindungen der Formel IV

$L^1L^2CH-(CH_2)_n-Br$ IV

Die Verbindungen der Formel IV sind zum Teil bekannt, zum Teil können sie ohne Schwierigkeiten nach Standardverfahren aus literaturbekannten Verbindungen hergestellt werden.

Die angegebenen Synthesemöglichkeiten sind nur beispielhaft für eine ganze Palette möglicher, dem Fachmann ohne weiteres bekannter Möglichkeiten So sind beispielsweise die Cyclopropylverbindungen der Formel I durch Umsetzung entsprechender Alkenderivate mit Dijodmethan/Zink - Kupfer - Paar (Simmons, Smith, J. Am. Chem. Soc. 81, 4256 (1959)) erhältlich. Diese Alkenderivate sowie weitere Synthesevarianten bzw. Synthesemöglichkeiten für Zwischenprodukte sind der Internationalen Patentanmeldung PCT/EP 90/01330 bzw. DE 40 25 550.6 zu entnehmen.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

$R'-L-CH_2CH_2-E-R''$ 4

R'-L-C = C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc- -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R'' voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R'' -F, -Cl, -NCS oder $-(O)_iCH_{3-(k+1)}F_kCl_l$, wobei i 0 oder 1 und k+l 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, $-CF_3$, $-OCHF_2$ oder $-OCF_3$ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R'' -CN; diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise

| | |
|---|---|
| Gruppe A: | 0 bis 90 %, vorzugsweise 20 bis 90 %, insbesondere 30 bis 90 % |
| Gruppe B: | 0 bis 80 %, vorzugsweise 10 bis 80 %, insbesondere 10 bis 65 % |
| Gruppe C: | 0 bis 80 %, vorzugsweise 5 bis 80 %, insbesondere 5 bis 50 % |

wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook

of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

Cyc = trans-1,4-Cyclohexylen, Phe = 1,4-Phenylen, Cp = Cyclopropyl, Cb = Cyclobutyl, K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste $C_nH_{2n+1}$ und $C_mH_{2m+1}$, sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten $R^1$, $R^2$, $L^1$ und $L^2$:

| Code für $R^1$, $R^2$, $L^1$, $L^2$ | $R^1$ | $R^2$ | $L^1$ | $L^2$ |
|---|---|---|---|---|
| nm | $C_nH_{2n+1}$ | $C_mH_{2m+1}$ | H | H |
| nOm | $C_nH_{2n+1}$ | $OC_mH_{2m+1}$ | H | H |
| nO.m | $OC_nH_{2n+1}$ | $C_mH_{2m+1}$ | H | H |
| nT | $C_nH_{2n+1}$ | CN | H | H |
| nN.F | $C_nH_{2n+1}$ | CN | H | F |
| nF | $C_nH_{2n+1}$ | F | H | H |
| nOF | $OC_nH_{2n+1}$ | F | H | H |
| nCl | $C_nH_{2n+1}$ | Cl | H | H |
| nF.F | $C_nH_{2n+1}$ | F | H | F |
| nOmFF | $C_nH_{2n+1}$ | $OC_mH_{2m+1}$ | F | F |
| nmF | $C_nH_{2n+1}$ | $C_mH_{2m+1}$ | F | H |
| $nCF_3$ | $C_nH_{2n+1}$ | $CF_3$ | H | H |
| $nOCF_3$ | $C_nH_{2n+1}$ | $OCF_3$ | H | H |
| $nOCF_2$ | $C_nH_{2n+1}$ | $OCHF_2$ | H | H |
| nS | $C_nH_{2n+1}$ | NCS | H | H |
| rVsN | $C_rH_{2r+1}-CH=CH-C_sH_{2s}-$ | CN | H | H |
| rEsN | $C_rH_{2r+1}-O-C_sH_{2s}-$ | CN | H | H |
| nNF | $C_nH_{2n+1}$ | CN | F | H |
| nAm | $C_nH_{2n+1}$ | $COOC_mH_{2m+1}$ | H | H |
| CprF.F | 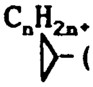 $(CH_2)_r-$ | F | H | F |
| CbrF.F | 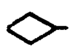 $(CH_2)_r-$ | F | H | F |

Tabelle A:

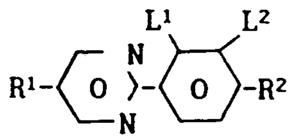

PYP

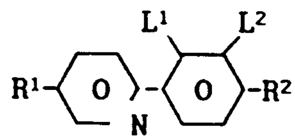

PYRP

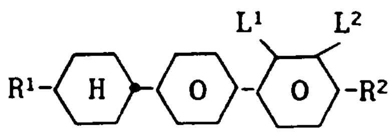

BCH

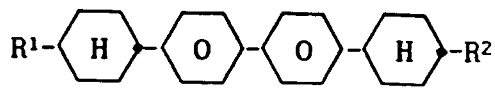

CBC

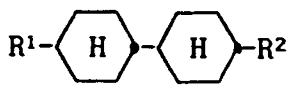

CCH

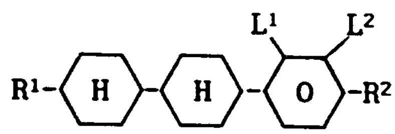

CCP

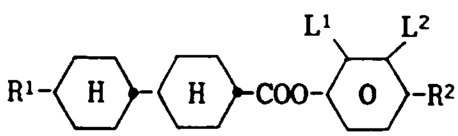

CP

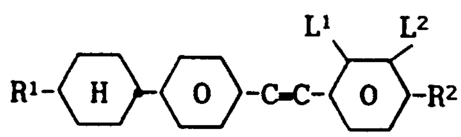

CPTP

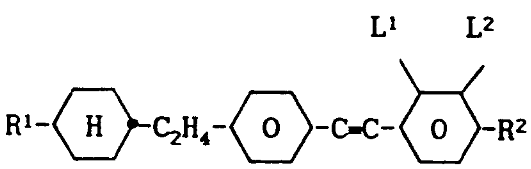

CEPTP

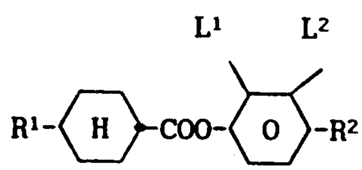

D

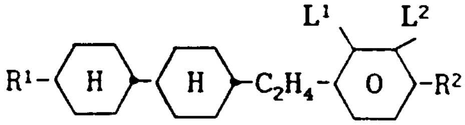

ECCP

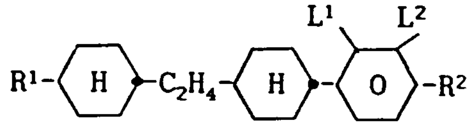

CECP

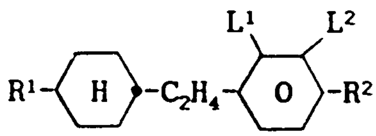

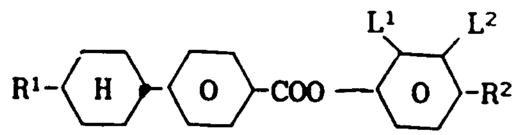

EPCH

HP

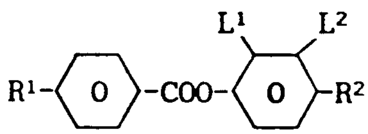

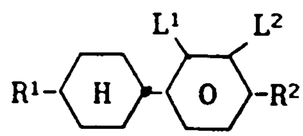

ME

PCH

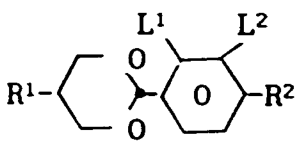

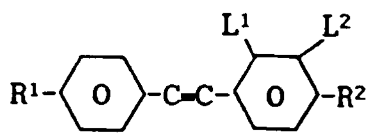

PDX

PTP

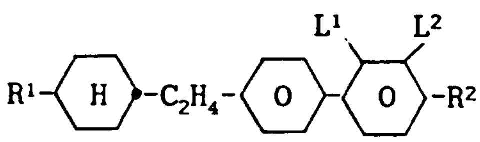

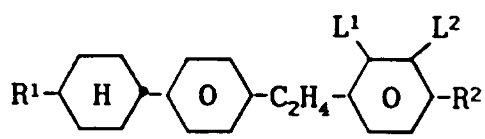

BECH

EBCH

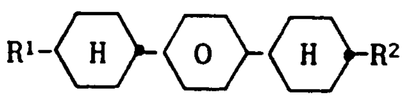

CPC

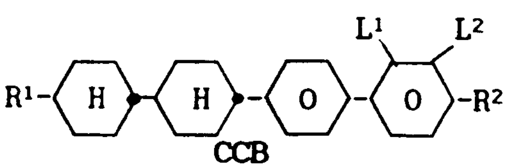

CCB

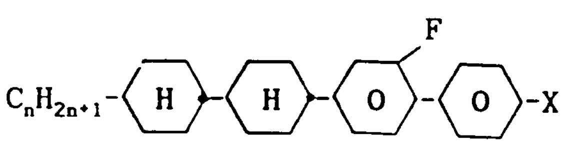

CCB-n.FX

Tabelle B:

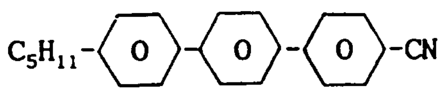

T15

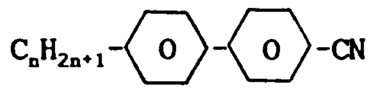

K3n

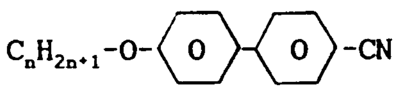

M3n

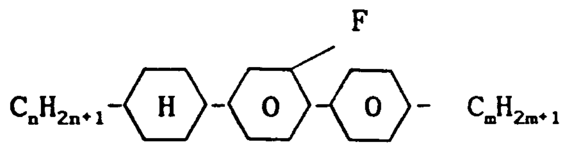

BCH-n.Fm

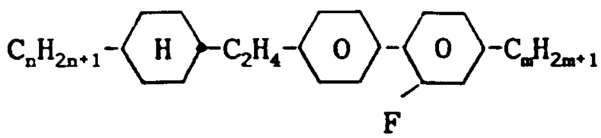

Inm

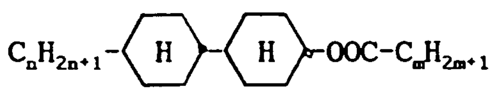

C-nm

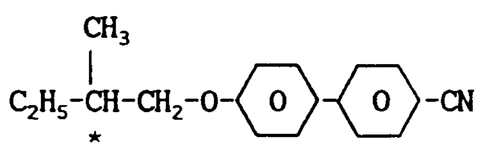

C15

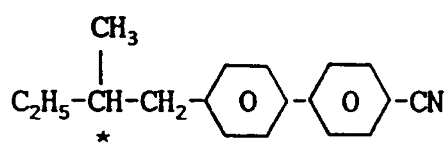

CB15

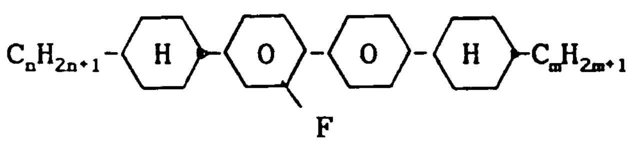

CBC-nmF

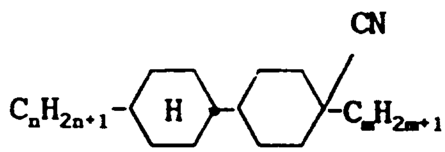

CCN-nm

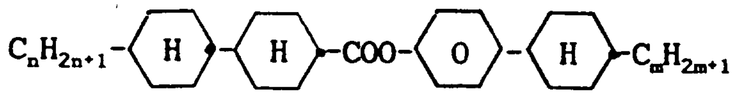

CCPC-nm

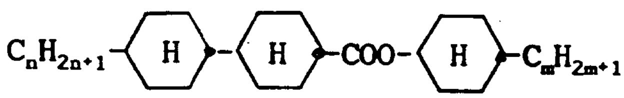

CH-nm

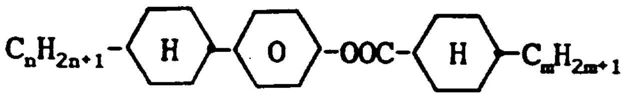

HD-nm

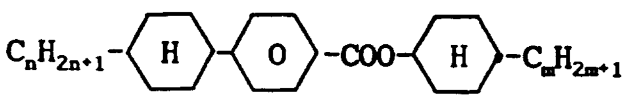

HH-nm

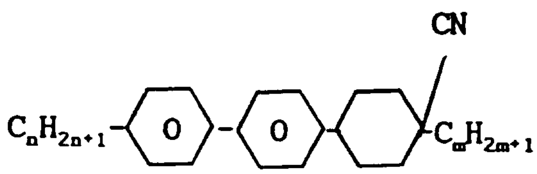

NCB-nm

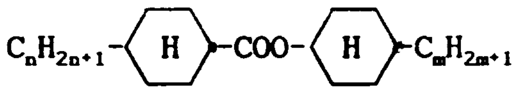

OS-nm

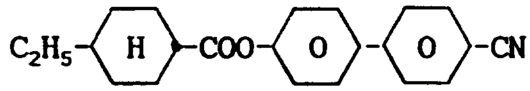

CHE

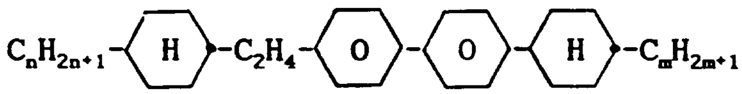

ECBC-nm

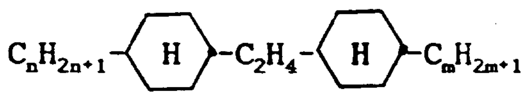

ECCH-nm

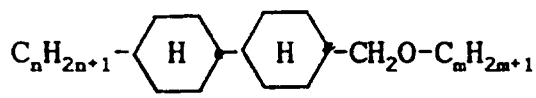

CCH-n1Em

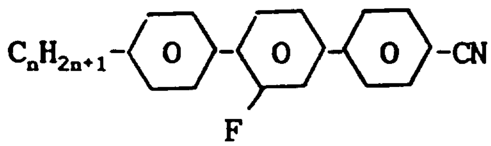

T-nFn

Beispiel 1:

Nach Hydrierung von 1-(2-Methylpropyl)-2-trans-4-(p-fluorphenyl)-cyclohexyl-ethen (hergestellt nach Wittig aus 3-Methylbutyr-aldehyd an Pd-C erhält man trans-1-(p-fluorphenyl)-4-(4-methylpentyl)-cyclohexan.

Beispiele 2 bis 103:

Analog Beispiel 1 erhält man aus den entsprechenden Aldehyden die folgenden Verbindungen:

| | $L^1L^2CH-$ | n | Q | X | Y | Z | A |
|---|---|---|---|---|---|---|---|
| (2) | $(CH_3)_2CH$ | 2 | $CH_2$ | F | H | H | '-' |
| (3) | $(CH_3)_2CH$ | 0 | $CH_2$ | F | H | H | '-' |
| (4) | Cp | 4 | $CH_2$ | F | H | H | '-' |
| (5) | Cb | 5 | $CH_2$ | F | H | H | '-' |
| (6) | $(CH_3)_2CH$ | 0 | $CH_2$ | F | F | H | '-' |
| (7) | $(CH_3)_2CH$ | 1 | $CH_2$ | F | F | H | '-' |
| (8) | $(CH_3)_2CH$ | 2 | $CH_2$ | F | F | H | '-' |
| (9) | Cp | 0 | $CH_2$ | F | F | H | '-' |
| (10) | Cb | 5 | $CH_2$ | F | F | H | '-' |
| (11) | $(C_2H_5)(CH_3)CH$ | 1 | $CH_2$ | F | F | H | '-' |
| (12) | $(CH_3)_2CH$ | 0 | $CH_2$ | Cl | H | H | '-' |
| (13) | $(CH_3)_2CH$ | 1 | $CH_2$ | Cl | H | H | '-' |
| (14) | $(CH_3)_2CH$ | 2 | $CH_2$ | Cl | H | H | '-' |

| | $L^1L^2CH$- | n | Q | X | Y | Z | A |
|---|---|---|---|---|---|---|---|
| (15) | Cp | 1 | $CH_2$ | Cl | H | H | '-' |
| (16) | Cb | 2 | $CH_2$ | Cl | H | H | '-' |
| (17) | $(C_2H_5)(CH_3)CH$ | 1 | $CH_2$ | Cl | H | H | '-' |
| (18) | $(CH_3)_2CH$ | 0 | $CH_2$ | Cl | F | H | '-' |
| (19) | $(CH_3)_2CH$ | 1 | $CH_2$ | Cl | F | H | '-' |
| (20) | $(CH_3)_2CH$ | 2 | $CH_2$ | Cl | F | H | '-' |
| (21) | Cp | 1 | $CH_2$ | Cl | F | H | '-' |
| (22) | Cb | 2 | $CH_2$ | Cl | F | H | '-' |
| (23) | $(C_2H_5)(CH_3)CH$ | 1 | $CH_2$ | Cl | F | H | '-' |
| (24) | $(CH_3)_2CH$ | 0 | $CH_2$ | $CF_3$ | H | H | '-' |
| (25) | $(CH_3)_2CH$ | 1 | $CH_2$ | $CF_3$ | H | H | '-' |
| (26) | $(CH_3)_2CH$ | 2 | $CH_2$ | $CF_3$ | H | H | '-' |
| (27) | Cp | 1 | $CH_2$ | $CF_3$ | H | H | '-' |
| (28) | Cb | 2 | $CH_2$ | $C_F$ | H | H | '-' |
| (29) | $(C_2H_5)(CH_3)CH$ | 1 | $CH_2$ | $CF_3$ | H | H | '-' |
| (30) | $(CH_3)_2CH$ | 0 | $CH_2$ | $OCF_3$ | H | H | '-' |
| (31) | $(CH_3)_2CH$ | 1 | $CH_2$ | $OCF_3$ | H | H | '-' |
| (32) | $(CH_3)_2CH$ | 2 | $CH_2$ | $OCF_3$ | H | H | '-' |
| (33) | Cp | 1 | $CH_2$ | $OCF_3$ | H | H | '-' |
| (34) | Cb | 2 | $CH_2$ | $OCF_3$ | H | H | '-' |
| (35) | $(C_2H_5)(CH_3)CH$ | 1 | $CH_2$ | $OCF_3$ | H | H | '-' |

| | R | n | Q | X | Y | Z | A |
|---|---|---|---|---|---|---|---|
| (36) | $(CH_3)_2CH$ | 2 | $CH_2$ | F | H | H | Cyc |
| (37) | $(CH_3)_2CH$ | 0 | $CH_2$ | F | H | H | Cyc |
| (38) | Cp | 0 | $CH_2$ | F | H | H | Cyc |
| (39) | Cb | 0 | $CH_2$ | F | H | H | Cyc |
| (40) | $(CH_3)_2CH$ | 0 | $CH_2$ | F | F | H | Cyc |
| (41) | $(CH_3)_2CH$ | 1 | $CH_2$ | F | F | H | Cyc |
| (42) | $(CH_3)_2CH$ | 2 | $CH_2$ | F | F | H | Cyc |
| (43) | Cp | 0 | $CH_2$ | F | F | H | Cyc |
| (44) | Cp | 0 | $CH_2$ | F | F | F | Cyc |
| (45) | Cb | 1 | $CH_2$ | F | F | H | Cyc |
| (46) | $(CH_3)_2CH$ | 0 | $CH_2$ | Cl | H | H | Cyc |
| (47) | $(CH_3)_2CH$ | 1 | $CH_2$ | Cl | H | H | Cyc |
| (48) | $(CH_3)_2CH$ | 2 | $CH_2$ | Cl | H | H | Cyc |
| (49) | Cp | 0 | $CH_2$ | Cl | H | H | Cyc |
| (50) | Cp | 0 | $CH_2$ | Cl | H | F | Cyc |
| (51) | Cb | 1 | $CH_2$ | Cl | H | H | Cyc |
| (52) | $(CH_3)_2CH$ | 0 | $CH_2$ | Cl | F | H | Cyc |
| (53) | $(CH_3)_2CH$ | 1 | $CH_2$ | Cl | F | H | Cyc |
| (54) | $(CH_3)_2CH$ | 2 | $CH_2$ | Cl | F | H | Cyc |
| (55) | Cp | 0 | $CH_2$ | Cl | F | H | Cyc |
| (56) | Cp | 0 | $CH_2$ | Cl | F | F | Cyc |
| (57) | Cb | 1 | $CH_2$ | Cl | F | H | Cyc |

| | R | n | Q | X | Y | Z | A |
|---|---|---|---|---|---|---|---|
| (58) | $(CH_3)_2CH$ | 0 | $CH_2$ | $CF_3$ | H | H | Cyc |
| (59) | $(CH_3)_2CH$ | 1 | $CH_2$ | $CF_3$ | H | H | Cyc |
| (60) | $(CH_3)_2CH$ | 2 | $CH_2$ | $CF_3$ | H | H | Cyc |
| (61) | Cp | 0 | $CH_2$ | $CF_3$ | H | H | Cyc |
| (62) | Cp | 0 | $CH_2$ | $CF_3$ | H | F | Cyc |
| (63) | Cb | 1 | $CH_2$ | $CF_3$ | H | H | Cyc |
| (64) | $(CH_3)_2CH$ | 0 | $CH_2$ | $OCF_3$ | H | H | Cyc |
| (65) | $(CH_3)_2CH$ | 1 | $CH_2$ | $OCF_3$ | H | H | Cyc |
| (66) | $(CH_3)_2CH$ | 2 | $CH_2$ | $OCF_3$ | H | H | Cyc |
| (67) | Cp | 0 | $CH_2$ | $OCF_3$ | H | H | Cyc |
| (68) | Cp | 0 | $CH_2$ | $OCF_3$ | H | F | Cyc |
| (69) | Cb | 1 | $CH_2$ | $OCF_3$ | H | H | Cyc |
| (70) | $(CH_3)_2CH$ | 2 | $CH_2$ | F | H | H | Phe |
| (71) | $(CH_3)_2CH$ | 0 | $CH_2$ | F | H | H | Phe |
| (72) | Cp | 0 | $CH_2$ | F | H | H | Phe |
| (73) | Cb | 0 | $CH_2$ | F | H | H | Phe |
| (74) | $(CH_3)_2CH$ | 0 | $CH_2$ | F | F | H | Phe |
| (75) | $(CH_3)_2CH$ | 1 | $CH_2$ | F | F | H | Phe |
| (76) | $(CH_3)_2CH$ | 2 | $CH_2$ | F | F | H | Phe |
| (77) | Cp | 0 | $CH_2$ | F | F | H | Phe |
| (78) | Cp | 0 | $CH_2$ | F | F | F | Phe |
| (79) | Cb | 1 | $CH_2$ | F | F | H | Phe |

| | R | n | Q | X | Y | Z | A |
|---|---|---|---|---|---|---|---|
| (80) | $(CH_3)_2CH$ | 0 | $CH_2$ | Cl | H | H | Phe |
| (81) | $(CH_3)_2CH$ | 1 | $CH_2$ | Cl | H | H | Phe |
| (82) | $(CH_3)_2CH$ | 2 | $CH_2$ | Cl | H | H | Phe |
| (83) | Cp | 0 | $CH_2$ | Cl | H | H | Phe |
| (84) | Cp | 0 | $CH_2$ | Cl | H | F | Phe |
| (85) | Cb | 1 | $CH_2$ | Cl | H | H | Phe |
| (86) | $(CH_3)_2CH$ | 0 | $CH_2$ | Cl | F | H | Phe |
| (87) | $(CH_3)_2CH$ | 1 | $CH_2$ | Cl | F | H | Phe |
| (88) | $(CH_3)_2CH$ | 2 | $CH_2$ | Cl | F | H | Phe |
| (89) | Cp | 0 | $CH_2$ | Cl | F | H | Phe |
| (90) | Cp | 0 | $CH_2$ | Cl | F | F | Phe |
| (91) | Cb | 1 | $CH_2$ | Cl | F | H | Phe |
| (92) | $(CH_3)_2CH$ | 0 | $CH_2$ | $CF_3$ | H | H | Phe |
| (93) | $(CH_3)_2CH$ | 1 | $CH_2$ | $CF_3$ | H | H | Phe |
| (94) | $(CH_3)_2CH$ | 2 | $CH_2$ | $CF_3$ | H | H | Phe |
| (95) | Cp | 0 | $CH_2$ | $CF_3$ | H | H | Phe |
| (96) | Cp | 0 | $CH_2$ | $CF_3$ | H | F | Phe |
| (97) | Cb | 1 | $CH_2$ | $CF_3$ | H | H | Phe |
| (98) | $(CH_3)_2CH$ | 0 | $CH_2$ | $OCF_3$ | H | H | Phe |
| (99) | $(CH_3)_2CH$ | 1 | $CH_2$ | $OCF_3$ | H | H | Phe |
| (100) | $(CH_3)_2CH$ | 2 | $CH_2$ | $OCF_3$ | H | H | Phe |
| (101) | Cp | 0 | $CH_2$ | $OCF_3$ | H | H | Phe |
| (102) | Cp | 0 | $CH_2$ | $OCF_3$ | H | F | Phe |
| (103) | Cb | 1 | $CH_2$ | $OCF_3$ | H | H | Phe |

Beispiel 104:

Nach Hydrierung von 1-Cyclobutyl-2-[trans-4-(3,4-difluorphenyl)-cyclohexyl]-ethen (hergestellt nach Wittig aus Cyclobutancarbaldehyd) an Pd-C erhält man 1-Cyclobutyl-2-[trans-4-(3,4-difluorphenyl)-cyclohexyl]-ethan.

Beispiele 105 bis 144:

Analog Beispiel 1 erhält man aus den entsprechenden Aldehyden die folgenden Verbindungen (Q = $CH_2$, n = 0,

$L^1L^2CH-$ = 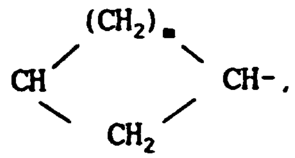

A = '-'):

| | m | X | Y | Z | R |
|---|---|---|---|---|---|
| (105) | 1 | F | F | H | $CH_3$ |
| (106) | 1 | F | F | H | $C_2H_5$ |
| (107) | 0 | F | F | H | H |
| (109) | 1 | F | H | H | H |
| (110) | 0 | F | H | H | $C_3H_7$ |
| (111) | 0 | F | H | H | n-$C_3H_7$ |
| (113) | 0 | Cl | H | H | $C_2H_5$ |
| (114) | 0 | Cl | H | H | n-$C_3H_7$ |
| (115) | 1 | Cl | H | H | H |

| | m | X | Y | Z | R |
|---|---|---|---|---|---|
| (117) | 0 | Cl | F | H | $C_2H_5$ |
| (118) | 0 | Cl | F | H | n-$C_3H_7$ |
| (119) | 1 | Cl | F | H | H |
| (121) | 0 | $CF_3$ | H | H | $C_2H_5$ |
| (122) | 0 | $CF_3$ | H | H | n-$C_3H_7$ |
| (123) | 1 | $CF_3$ | H | H | H |
| (125) | 0 | $OCF_3$ | H | H | $C_2H_5$ |
| (126) | 0 | $OCF_3$ | H | H | n-$C_3H_7$ |
| (127) | 1 | $OCF_3$ | H | H | H |
| (129) | 0 | $OCHF_2$ | H | H | $C_2H_5$ |
| (130) | 0 | $OCHF_2$ | H | H | n-$C_3H_7$ |
| (131) | 1 | $OCHF_2$ | H | H | H |
| (133) | 0 | CN | H | H | $C_2H_5$ |
| (134) | 0 | CN | H | H | n-$C_3H_7$ |
| (135) | 1 | CN | H | H | H |
| (137) | 0 | CN | F | H | $C_2H_5$ |
| (138) | 0 | CN | F | H | n-$C_3H_7$ |
| (139) | 1 | CN | F | H | H |

| | m | X | Y | Z | R |
|---|---|---|---|---|---|
| (141) | 0 | CN | F | F* | $C_2H_5$ |
| (142) | 0 | CN | F | F* | n-$C_3H_7$ |
| (143) | 1 | CN | F | F* | H |

* Z in ortho-Position zu X

Beispiel 145

Nach Umsetzung von

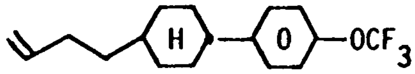

mit $CH_2J_2$ und Zn-Cu-Paar nach Simmons-Smith erhält man

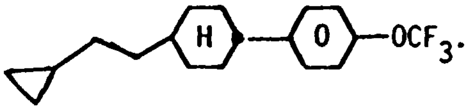

Beispiel A:

| | |
|---|---|
| 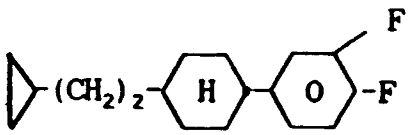 | 8 % |
| PCH-6F | 6 % |
| PCH-7F | 5 % |
| 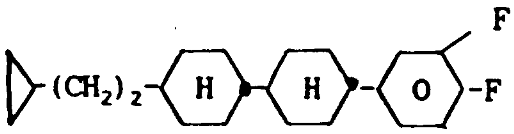 | 4 % |
| 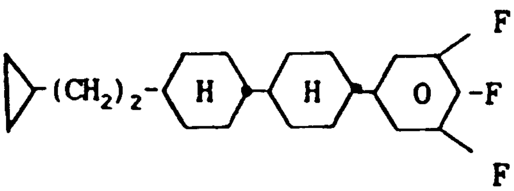 | 4 % |
| CCP-2 $OCF_3$ | 6 % |
| CCP-3 $OCF_3$ | 9 % |
| CCP-4 $OCF_3$ | 6 % |
| CCP-5 $OCF_3$ | 9 % |
| 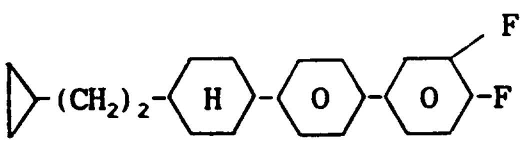 | 8 % |
| 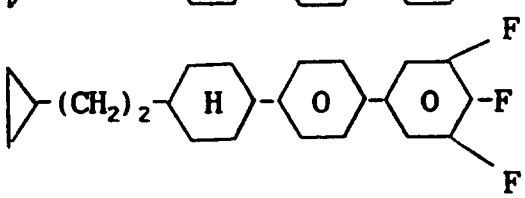 | 4 % |
| CP-3F | 7 % |
| CP-5F | 4 % |
| CCB-3F.F | 10 % |
| CCB-5F.F | 10 % |

$V_{th}$ = 1,73 V

Klärpunkt = 83°C

Beispiel B:

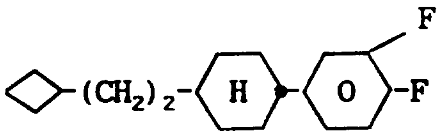 8 %

PCH-6F 6 %

PCH-7F 5 %

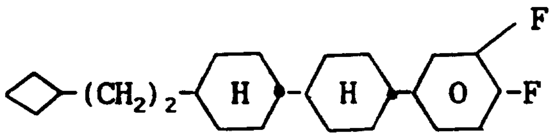 4 %

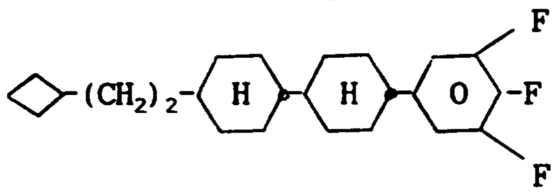 4 %

CCP-2 $OCF_3$ 6 %

CCP-3 $OCF_3$ 9 %

CCP-4 $OCF_3$ 6 %

CCP-5 $OCF_3$ 9 %

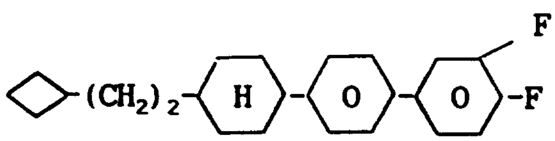 8 %

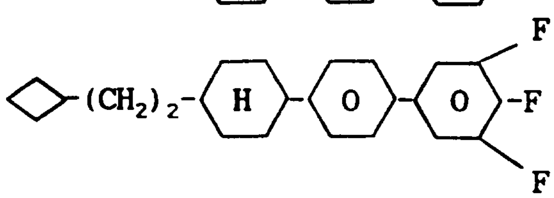 4 %

CP-3F 7 %

CP-5F 4 %

CCB-3F.F 10 %

CCB-5F.F 10 %

$V_{th}$ = 1,81 V

Klärpunkt = 86°C

Beispiel C:

| | |
|---|---|
| 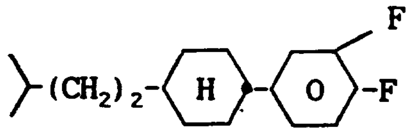 | 8 % |
| PCH-6F | 6 % |
| PCH-7F | 5 % |
| 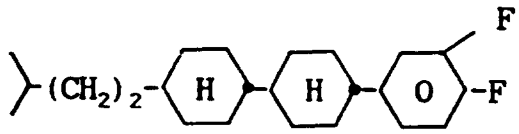 | 4 % |
| 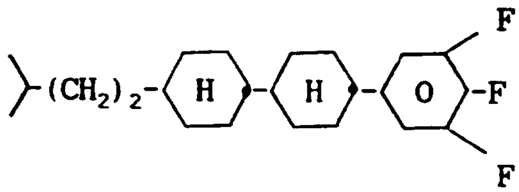 | 4 % |
| CCP-2 $OCF_3$ | 6 % |
| CCP-3 $OCF_3$ | 9 % |
| CCP-4 $OCF_3$ | 6 % |
| CCP-5 $OCF_3$ | 9 % |
| 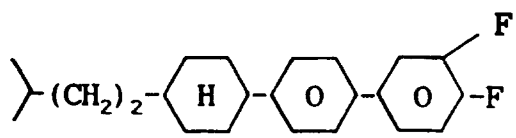 | 8 % |
| 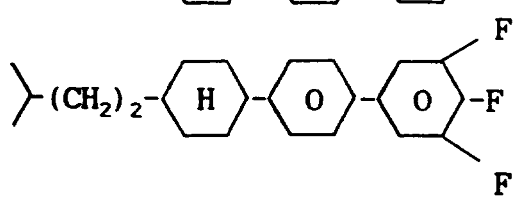 | 4 % |
| CP-3F | 7 % |
| CP-5F | 4 % |
| CCB-3F.F | 10 % |
| CCB-5F.F | 10 % |

$V_{th}$ = 1,68 V

Klärpunkt = 76°C

## Patentansprüche

**1.** Phenylcyclohexane der Formel I

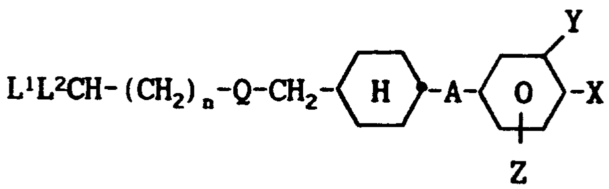

I

worin

L¹ und L² — jeweils $CH_3$ oder einer der Reste $L^1$ und $L^2$ auch $C_2H_5$ oder $L^1L^2CH$- auch

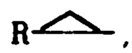

,

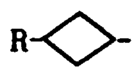

,

wobei R H oder geradkettiges Niederalkyl mit bis zu 5 C-Atomen bedeutet,

| | |
|---|---|
| n | 0 bis 5, |
| Q | -O- oder $-CH_2-$, |
| A | trans-1,4-Cyclohexylen, 1,4-Phenylen, 3-Fluor-1,4-phenylen oder eine Einfachbindung, |
| X | F, Cl, $-CF_3$, -CN, $-OCF_3$ oder $-OCHF_2$ und |
| Y und Z | jeweils unabhängig voneinander H oder F bedeuten. |

**2.** Phenylcyclohexane nach Anspruch 1, dadurch gekennzeichnet, daß X F, Cl, $-CF_3$, $-OCHF_2$ oder $-OCF_3$ ist.

**3.** Phenylcyclohexane nach Anspruch 1 oder 2, dadurch gekennzeichnet, daß Y = Z = H.

**4.** Phenylcyclohexane nach Anspruch 1 oder 2, dadurch gekennzeichnet, daß Y = F und Z = H oder F.

**5.** Phenylcyclohexane nach mindestens einem der Ansprüche 1 bis 4, gekennzeichnet durch die Formel Ia

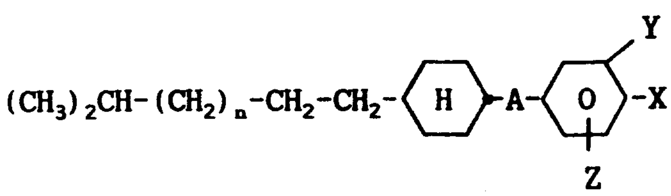

Ia

**6.** Phenylcyclohexane nach mindestens einem der Ansprüche 1 bis 4, gekennzeichnet durch die Formel Ib

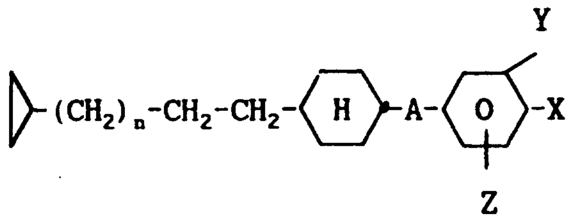

Ib

7. Phenylcyclohexane nach mindestens einem der Ansprüche 1 bis 4, gekennzeichnet durch die Formel Ic

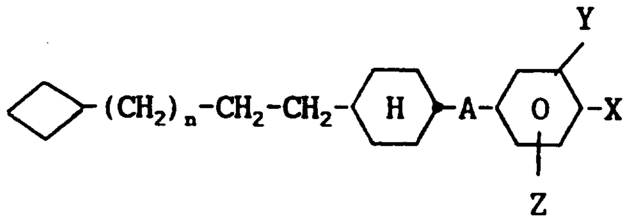

Ic

8. Verwendung der Phenylcyclohexane der Formel I nach Anspruch 1 als Komponenten flüssigkristalliner Medien für elektrooptische Anzeigen.

9. Flüssigkristallines Medium für elektrooptische Anzeigen mit mindestens zwei flüssigkristallinen Komponenten, dadurch gekennzeichnet, daß mindestens eine Komponente ein Phenylcyclohexan der Formel I nach Anspruch 1 ist.

10. Elektrooptische Anzeige auf der Basis einer Flüssigkristallzelle, dadurch gekennzeichnet, daß die Flüssigkristallzelle ein Medium nach Anspruch 9 enthält.

## Claims

1. Phenylcyclohexanes of the formula I

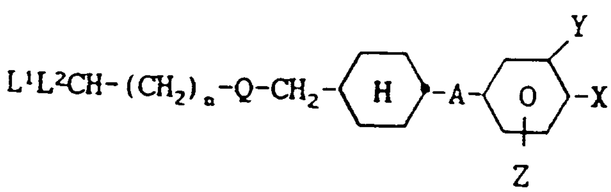

I

in which

$L^1$ and $L^2$ are each $CH_3$, or one of the radicals $L^1$ and $L^2$ is alternatively $C_2H_5$, or $L^1L^2CH$- is alternatively

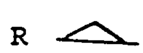 , 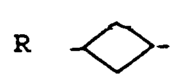 ,

where R is H or straight-chain lower alkyl having up to 5 carbon atoms,

n is 0 to 5,

Q is -O- or $-CH_2-$,

A is trans-1,4-cyclohexylene, 1,4-phenylene, 3-fluoro-1,4-phenylene or a single bond,

| | |
|---|---|
| X | is F, Cl, $-CF_3$, -CN, $-OCF_3$ or $-OCHF_2$ and |
| Y and Z | are each, independently of one another, H or F. |

**2.** Phenylcyclohexanes according to Claim 1, characterised in that X is F, Cl, $-CF_3$, $-OCHF_2$ or $-OCF_3$.

**3.** Phenylcyclohexanes according to Claim 1 or 2, characterised in that Y = Z = H.

**4.** Phenylcyclohexanes according to Claim 1 or 2, characterised in that Y = F and Z = H or F.

**5.** Phenylcyclohexanes according to at least one of Claims 1 to 4, characterised by the formula Ia

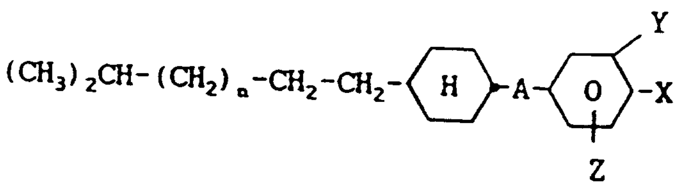

Ia

**6.** Phenylcyclohexanes according to at least one of Claims 1 to 4, characterised by the formula Ib

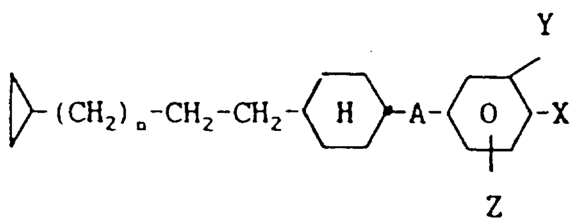

Ib

**7.** Phenylcyclohexanes according to at least one of Claims 1 to 4, characterised by the formula Ic

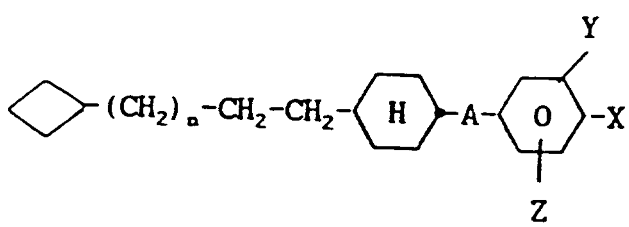

Ic

**8.** Use of the phenylcyclohexanes of the formula I according to Claim 1 as components of liquid-crystalline media for electrooptical displays.

**9.** Liquid-crystalline medium for electrooptical displays containing at least two liquid-crystalline components, characterised in that at least one component is a phenylcyclohexane of the formula I according to Claim 1.

**10.** Electrooptical display based on a liquid-crystal cell, characterised in that the liquid-crystal cell contains a medium according to Claim 9.

## Revendications

**1.** Phénylcyclohexanes de formule I

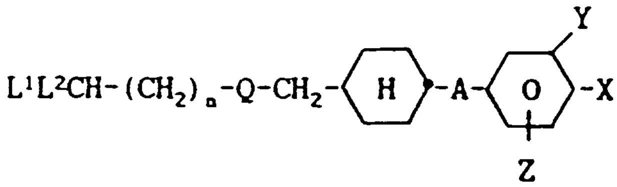

I

où
$L^1$ et $L^2$ représentent $CH_3$ ou l'un des restes $L^1$ et
$L^2$ représente également $C_2H_5$ ou $L^1L^2CH$- représente également

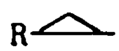 , 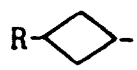 ,

R représentant H ou un alkyle inférieur à chaîne droite ayant jusqu'à 5 atomes de C,
n va de 0 à 5,
Q représente -O- ou $-CH_2-$,
A représente le trans-1,4-cyclohexylène, le 1,4-phénylène, le 3-fluoro-1,4-phénylène ou une liaison simple,
X représente F, Cl, $-CF_3$, -CN, $-OCF_3$ ou $-OCHF_2$ et
Y et Z représentent, indépendamment l'un de l'autre, H ou F.

**2.** Phénylcyclohexanes selon la revendication 1, caractérisés en ce que X est Y, Cl, $-CF_3$, $-OCHF_2$ ou $-OCF_3$.

**3.** Phénylcyclohexanes selon la revendication 1 ou 2, caractérisés en ce que Y = Z = H.

**4.** Phénylcyclohexanes selon la revendication 1 ou 2, caractérisés en ce que Y = F et Z = H ou F.

**5.** Phénylcyclohexanes selon au moins l'une des revendications 1 à 4, caractérisés en ce qu'ils répondent à la formule Ia

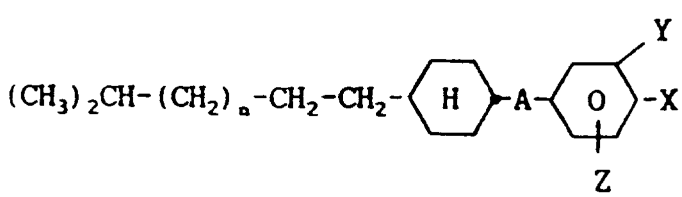

Ia

**6.** Phénylcyclohexanes selon au moins l'une des revendications 1 à 4, caractérisés en ce qu'ils répondent à la formule Ib

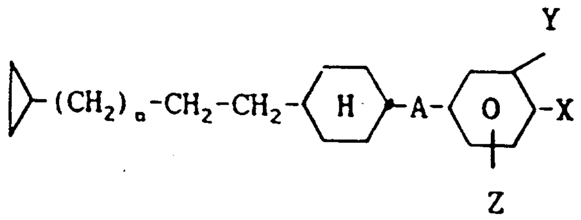

Ib

7. Phénylcyclohexanes selon au moins l'une des revendications 1 à 4, caractérisés en ce qu'ils répondent à la formule Ic

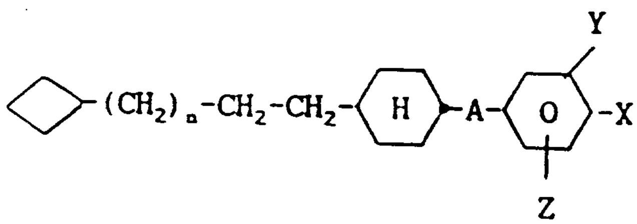

Ic

8. Utilisation des phénylcyclohexanes de formule I selon la revendication 1, comme composant de milieux à cristaux liquides pour affichages électro-optiques.

9. Milieu à cristaux liquides pour affichages électro-optiques contenant au moins deux composants à cristaux liquides, caractérisé en ce qu'au moins l'un des composants est un phénylcyclohexane de formule I selon la revendication 1.

10. Affichage électro-optique à base d'une cellule à cristaux liquides, caractérisé en ce que la cellule à cristaux liquides contient un milieu selon la revendication 9.